# FASCICULE DE BREVET EUROPEEN

(11) **EP 1 171 574 B1**
(45) Date de publication et mention de la délivrance du brevet: **03.11.2004**
(21) Numéro de dépôt: 00920796.0
(22) Date de dépôt: 14.04.2000
(51) Int. Cl.: C12N 1/14, C12P 7/42, C12P 7/24

(54) **PROCEDE D'OBTENTION DE CULTURES D'ASPERGILLUS NIGER ET LEURS UTILISATIONS POUR LA PRODUCTION D'ACIDE FERULIQUE ET D'ACIDE VANILLIQUE**
VERFAHREN ZUR GEWINNUNG VON ASPERGILLUS NIGER KULTUREN UND IHRE VERWENDUNG ZUR HERSTELLUNG VON FERULASÄURE UND VANILLINSÄURE
METHOD FOR OBTAINING ASPERGILLUS NIGER CULTURES AND THEIR USES FOR PRODUCING FERULIC ACID AND VANILLIC ACID

(30) Priorité: 14.04.1999 FR 9904644
(43) Date de publication de la demande: 16.01.2002
(73) Titulaire: INSTITUT NATIONAL DE LA RECHERCHE AGRONOMIQUE (INRA), 75007 Paris Cédex 07 (FR)
(72) Inventeur: Bonnin, Estelle, 44700 Orvault (FR); Lesage-Meessen, Laurence, 13008 Marseille (FR); Stentelaire, Christelle, 13127 Vitrolles (FR); Asther, Marcel, 13600 La Ciotat (FR); Thibault, Jean-François, 44700 Orvault (FR)
(74) Mandataire: Vialle-Presles, Marie José
(86) Numéro de dépôt international: PCT/FR2000/000966
(87) Numéro de publication internationale: WO 2000/061721

(56) Documents cités:
- KROON P A ET AL: "RELEASE OF FERULIC ACID FROM SUGAR-BEET PULP BY USING ARABINANASE, ARABINOFURANOSIDASE AND AN ESTERASE FROM ASPERGILLUS NIGER" BIOTECHNOLOGY AND APPLIED BIOCHEMISTRY,US,ACADEMIC PRESS, vol. 23, no. 3, 1996, page 263-267 XP000603360 ISSN: 0885-4513
- LESAGE-MEESEN L ET AL: "Fungal tranformation of ferulic acid from sugar beet pulp to natural vanillin" JOURNAL OF THE SCIENCE OF FOOD AND AGRICULTURE., vol. 79, mars 1999 (1999-03), pages 487-490, XP002124793 ELSEVIER APPLIED SCIENCE PUBLISHERS. BARKING., GB ISSN: 0022-5142
- MICARD V ET AL: "STUDIES ON ENZYMIC RELEASE OF FERULIC ACID FROM SUGAR-BEET PULP" LEBENSMITTEL WISSENSCHAFT UND TECHNOLOGIE,GB,LONDON, vol. 27, no. 1, 1994, page 59-66 XP000603509

## Description

La présente Invention est relative à l'obtention d'acide férulique et d'acide vanillique par bioconversion.

La vanilline, qui est actuellement l'arôme le plus utilisé dans les industries agro-alimentaires, peut avantageusement être produite par bioconversion d'acide férulique ou d'acide vanillique (qui est lui-même un produit de bioconversion de l'acide férulique), à l'aide de champignons filamenteux.

Ainsi, la Demande de brevet européen 453 368 au nom de la Société PERNOD-RICARD, décrit la production de vanilline naturelle par bioconversion d'acide férulique ou d'acide vanillique, en présence d'un champignon filamenteux du groupe des Basidiomycètes, *Pycnaporus cinnabarinus*. La Demande PCT WO/96/08576 au nom de l'INRA, décrit un procédé de bioconversion en 2 étapes, permettant d'obtenir un rendement plus élevé. Dans la première étape, l'acide férulique est converti en acide vanillique par un champignon filamenteux (Ascomycète, Basidiomycète ou Actinomycète) ; l'acide vanillique obtenu est ensuite converti en vanilline par un Basidiomycète.

L'acide férulique, qui constitue le produit de départ de ces procédés de bioconversion, est l'un des composés phénoliques majeurs de la paroi cellulaire des végétaux. Il a été décrit chez les monocotylédones, notamment les céréales (blé, maïs,...), ainsi que chez les dicotylédones de la famille des Chénopodiacées (betterave, épinard,...). Il est généralement estérifié aux polysaccharides de la paroi végétale, par l'intermédiaire de l'arabinose ou du galactose dans les pectines de betterave, ou de l'arabinose dans les arabinoxylanes de céréales.

L'acide férulique est présent dans divers co-produits agricoles : par exemple, les sons de blé (résidus de la meunerie) et de maïs (résidus de la semoulerie) et les pulpes de betterave (résidus de l'industrie du sucre), contiennent de 0,6 à 4% d'acide férulique. Ces produits constituent des sources potentielles facilement disponibles et peu coûteuses d'acide férulique.

Bien qu'il soit théoriquement envisageable, dans le cadre de la mise en oeuvre des procédés de bioconversion décrits dans la Demande EP 0 453 368 ou la demande PCT WO/96/08576 de produire de l'acide vanillique directement à partir d'acide férulique estérifié aux polysaccharides, le taux de bioconversion dans ces conditions est négligeable.

L'acide férulique doit donc être préalablement libéré par des férulate estérases. Selon la nature du polysaccharide portant l'acide férulique, la liaison acide férulique-ose est différente : l'acide férulique forme une liaison ester avec le O-5 de l'arabinose dans les arabinoxylanes de céréales, alors que dans le cas des pectines de betterave, l'acide férulique est porté soit par le O-2 de l'arabinose, soit dans une moindre mesure par le O-6 du galactose. La libération de l'acide férulique impliquera donc des enzymes différentes selon la nature de la liaison à rompre : les principales férulate estérases mises en évidence chez *A. niger* sont indiquées dans le Tableau I ci-dessous.

**Tableau I**

| Enzyme | Origine | Substrat préférentiel |
|---|---|---|
| FAE I | *Aspergillus niger* | arabinose féruloylé en O-2 /galactose féruloylé en O-6 |
| FAEII | *Aspergillus niger* | arabinose féruloylé en O-5 |
| FAEIII | *Aspergillus niger* | arabinose féruloylé en O-5 |
| CinnAE | *Aspergillus niger* | arabinose féruloylé en O-2 |

En outre, les férulate estérases n'agissent pas directement sur les polysaccharides des parois végétales : les liaisons existant entre les oses au sein des polysaccharides pariétaux doivent préalablement être rompues pour que l'acide férulique puisse être libéré par les férulate estérases. Etant donné la diversité des polysaccharides pariétaux, la rupture de ces liaisons nécessite un grand nombre d'activités enzymatiques différentes dont les principales sont : polygalacturonase, rhamnogalacturonase, arabinanase, galactanase, xylanase et glucanase. Les produits d'hydrolyse libérés par ces enzymes sont à leur tour dégradés par des osidases (arabinofuranosidase et galactosidase) et l'acide férulique est libéré par des férulate estérases.

Des mélanges enzymatiques permettant de libérer l'acide férulique présent dans les polysaccharides pariétaux des pulpes de betterave ou des sons de céréales sont commercialisés. Toutefois, ces mélanges enzymatiques n'ont pas une activité férulate estérase suffisante, et doivent être supplémentés en férulate estérases extraites de milieux de culture microbiens. En outre, une fois l'acide férulique libéré sa purification implique de nombreuses étapes, longues et coûteuses, de séparation liquide/solide et liquide/liquide.

Or les Inventeurs sont maintenant parvenus à induire chez *Aspergillus niger* la production d'enzymes à large spectre d'activité, permettant non seulement une libération efficace de l'acide férulique, mais en outre la production directe d'acide vanillique naturel à partir de co-produits agricoles.

La présente invention a pour objet un procédé d'obtention de cultures d'*Aspergillus niger* à large spectre d'activité enzymatique, caractérisé en ce qu'il comprend la mise en culture d'au moins une souche d'*Aspergillus niger* en présence d'au moins une source carbonée inductrice choisie dans le groupe constitué par :
- la pulpe de betterave ou au moins une de ses fractions solubles riches en oligosaccharides féruloylés, susceptibles d'être obtenues par hydrolyse acide ;
- un son de céréale, notamment de maïs, ou un mélange de sons de différentes céréales, ou au moins une de ses fractions solubles riches en oligosaccharides féruloylés, susceptibles d'être obtenues par autoclavage dudit son ou dudit mélange.

Selon un mode de mise en oeuvre préféré de la présente invention, ladite source carbonée inductrice est présente dans ledit milieu de culture à une concentration comprise entre 1 et 50 grammes (poids sec), et de préférence entre 2,5 et 30 grammes par litre de milieu de culture.

Selon un autre mode de mise en oeuvre préféré de la présente invention, la culture d'*Aspergillus niger* comprend au moins la souche CNCM I-1472, déposée le 31 août 1994 auprès de la CNCM (Collection Nationale de Cultures de Micro-organismes, 26 rue du Docteur Roux, Paris).

La présente invention a aussi pour objet :
- un procédé d'obtention d'une préparation enzymatique à large spectre d'activité, caractérisé en ce qu'il comprend la mise en culture d'au moins une souche d'*Aspergillus niger* selon le procédé défini ci-dessus, et la récupération du surnageant de culture ;
- une préparation enzymatique constituée par ledit surnageant de culture.

La récupération du surnageant de culture peut s'effectuer par tous moyens connus en eux-mêmes, tels que centrifugation ou filtration, qui permettent de séparer les cellules d'*Aspergillus niger* du milieu de culture. Une préparation enzymatique conforme à l'invention peut être constituée par le surnageant lui-même, ou par un concentré dudit surnageant, obtenu par exemple par ultrafiltration ou par lyophilisation.

La présente invention a aussi pour objet un procédé de production d'acide férulique libre à partir d'un substrat féruloylé, lequel procédé est caractérisé en ce qu'il comprend la mise en contact dudit substrat avec au moins une culture d'*Aspergillus niger* préalablement obtenue selon le procédé conforme à l'invention, ou avec au moins une préparation enzymatique conforme à l'invention, dans des conditions permettant la libération de l'acide férulique par les enzymes présentes dans ladite culture ou ladite préparation enzymatique.

Au sens de la présente invention, on entend par : « substrat féruloylé », tout produit contenant ou constitué par au moins un polysaccharide féruloylé et/ou au moins un oligosaccharide féruloylé. Il s'agit notamment de tout substrat d'origine végétale comprenant des polysaccharides pariétaux féruloylés et/ou des oligosaccharides féruloylés.

Dans le cas où le substrat végétal comprend principalement des polysaccharides pariétaux insolubles, on pourra avantageusement les rendre plus accessibles à l'hydrolyse enzymatique, en les soumettant préalablement à un traitement chimique tel qu'une hydrolyse acide.ou alcaline, et/ou à un traitement physique tel que l'autoclavage ou la cuisson-extrusion.

A titre de substrats végétaux particulièrement avantageux pour la mise en oeuvre de la présente invention, on citera notamment :
- de la pulpe de betterave ou ses fractions solubles riches en oligosaccharides féruloylés, susceptibles d'être obtenues par hydrolyse acide ;
- un son de céréale, notamment de maïs, ou un mélange de sons de différentes céréales, ou leurs fractions solubles riches en oligosaccharides féruloylés, susceptibles d'être obtenues par autoclavage.

La mise en contact du substrat féruloylé avec au moins une souche d'*Aspergillus niger* préalablement cultivée conformément à l'invention peut s'effectuer en ajoutant au milieu de culture d'*Aspergillus niger* une quantité dudit substrat féruloylé correspondant par exemple à un apport de 0,1 à 50 g d'acide férulique par litre de milieu de culture. De préférence, la quantité de substrat féruloylé ajoutée au milieu de culture correspond à un apport de 1 à 20 g, et avantageusement à un apport de 5 à 15 g d'acide férulique par litre de milieu de culture.

La mise en contact du substrat féruloylé avec au moins une préparation enzymatique conforme à l'invention peut s'effectuer en mélangeant ladite préparation enzymatique avec ledit substrat féruloylé, par exemple dans des proportions correspondant à un apport, par ledit substrat, de 0,1 à 40 g d'acide férulique pour 1 gramme de protéines totales de préparation enzymatique. De préférence, les proportions du mélange correspondent à un apport de 0,2 à 10 g et avantageusement de 0,5 à 5 g d'acide férulique, pour 1 gramme de protéines totales de la préparation enzymatique.

La quantité de substrat féruloylé ajoutée au milieu de culture ou à la préparation enzymatique varie notamment en fonction de la nature dudit substrat et de sa teneur initiale en acide férulique estérifié. Cette teneur peut être facilement déterminée par toute méthode connue en elle-même de l'homme de l'art, par exemple par la méthode décrite par SAULNIER et al. [Carbohydrate Research, 272, 241-253, (1995)].

Ledit substrat féruloylé peut être ajouté en une seule fois, en plusieurs fois par ajouts successifs, ou bien en continu.

Avantageusement, pour la mise en oeuvre du procédé conforme à l'invention, on utilisera une culture d'*Aspergillus niger* ou une préparation enzymatique produites en présence d'une source carbonée inductrice comprenant de la pulpe de betterave ou au moins une de ses fractions riches en oligosaccharides féruloylés susceptibles d'être obtenues par hydrolyse acide, et un substrat féruloylé comprenant au moins un son de céréale, notamment de maïs, ou au moins une de ses fractions riches en oligosaccharides féruloylés, obtenues par autoclavage.

L'acide férulique produit dans ces conditions peut, si on le souhaite, être recueilli à partir du milieu de culture. Cependant, il est particulièrement avantageux d'effectuer directement la bioconversion de l'acide férulique en acide vanillique, par la même culture d'*Aspergillus niger,* dont les cellules possèdent les enzymes intracellulaires nécessaires à cette bioconversion.

L'acide férulique ou l'acide vanillique obtenus conformément à l'invention pourront être utilisés tels quels, par exemple comme antioxydants, ou bien pourront être utilisés comme précurseurs de vanilline dans des procédés de bioconversion tels que ceux décrits dans la Demande EP 0 453 368 ou la Demande PCT WO/96/08576).

La présente Invention sera mieux comprise à l'aide du complément de description qui va suivre, qui se réfère à des exemples de mise en oeuvre du procédé conforme à l'Invention.

Il doit être bien entendu toutefois que ces exemples sont donnés uniquement à titre d'illustration de l'objet de l'Invention dont ils ne constituent en aucune manière une limitation.

### EXEMPLE 1 :ACTIVITÉ ENZYMATIQUE D'A. NIGER I-1472 CULTIVÉ SUR PULPE DE BETTERAVE :

### A) Réalisation des cultures d'Aspergillus niger I-1472

La souche d'*Aspergillus niger* déposée le 31 Août 1994 auprès de la Collection Nationale de Cultures de Micro-organismes, sous le numéro I-1472, a été mise en culture en présence de co-produits agricoles ou de leurs fractions comme sources carbonées inductrices d'enzymes d'intérêt.

La composition du milieu de culture est la suivante :

| | |
|---|---|
| Source carbonée inductrice | 15,00 g/L |
| Maltose | 2,50 g/L |
| Tartrate diammonium | 1,842 g/L |
| KH₂PO₄ | 0,20 g/L |
| CaCl₂, 2 H₂O | 0,0132 g/L |
| MgSO₄, 7 H₂O | 0,50 g/L |
| Extrait de levure | 0,50 g/L |
| Tween 80 | 0,50 g/L |

De la pulpe de betterave est utilisée comme source de carbone inductrice.

La composition de la pulpe de betterave (poids sec) est la suivante :

| | |
|---|---|
| Rhamnose | 24 mg/g |
| Arabinose | 209 mg/g |
| Xylose | 17 mg/g |
| Galactose | 51 mg/g |
| Glucose | 211 mg/g |
| Acides uroniques | 211 mg/g |
| Acide férulique | 8 mg/g |
| Protéines | 113 mg/g |
| Cendres | 36 mg/g |

Une culture témoin est réalisée sur maltose (20 g/L) comme seule source de carbone. Le milieu est stérilisé par autoclavage 20 minutes à 120°C. Les cultures se déroulent en fioles de 500 mL contenant 200 mL de milieu. L'inoculation est effectuée par des conidiospores (2,10⁵ spores/mL). Après inoculation, les cultures sont incubées à 30°C et soumises à une agitation de 120 tours/minute.

La production d'enzymes au cours de la culture est suivie par le dosage des activités enzymatiques produites. Pour procéder à ce dosage, un aliquot du milieu de culture d'environ 10 mL est prélevé stérilement chaque jour. Cet aliquot est ensuite filtré sur fibres de verre, et les activités enzymatiques vis-à-vis de différents substrats sont dosées.

### B) Dosage des activités enzymatiques

### 1) Enzymes de dépolymérisation

Les activités enzymatiques sont mesurées sur différents polysaccharides purs : galacturonane, carboxyméthylcellulose, xylane, galactane de type I, arabinane, rhamnogalacturonane.

Le milieu réactionnel contient 0,9 mL de solution de substrat à 1 g/L dans le tampon acétate 50 mmoles/L pH 4,5 et 0,1 mL de filtrat de culture. Le mélange est incubé 10 minutes à 40°C. Les extrémités réductrices libérées sont dosées en microplaques par le sulfate de cuivre [NELSON, J. Biol. Chem. 153, 375-380 (1944) ; STURGEON, Methods in Plant Biochemistry, 2, 1-37 (1990)]. L'ose constitutif du polysaccharide est utilisé comme standard : acide galacturonique pour les dosages sur galacturonane et rhamnogalacturonane, glucose pour les dosages sur carboxyméthylcellulose, xylose pour les dosages sur xylane, galactose pour les dosages sur galactane et arabinose pour les dosages sur arabinane.

### 2) Activités osidases

Les activités osidases sont mesurées sur *para*-nitrophényl-β-D-galactopyranoside et *para*-nitro-phényl-α-L-arabinofuranoside (SIGMA).

Le milieu réactionnel contient 0,1 mL de solution de substrat à 4 mmoles/L dans le tampon acétate 50 mmoles/L pH 4,5 et 0,1 mL de filtrat du surnageant de culture. Le mélange est incubé 20 minutes à 40°C. 0,6 mL de carbonate de sodium sont alors ajoutés pour inhiber l'activité enzymatique et permettre le développement de la réaction colorimétrique. La concentration en *para* nitrophénol libéré est calculée à partir de la densité optique du mélange lue à λ = 400 nm.

### 3) Activités férulate estérase

Les activités des férulate estérases sont mesurées sur différents oligomères féruloylés : 5-O-(transféruloyl)-L-Araf (détermination de l'activité FA) et O-β-D-Xyln(1->2)-[5-O-(transféruloyl)-α-L-Araf] (détermination de l'activité XFA) isolés de son de maïs [SAULNIER et al., Carbohydrate Research, 272, 241-253, (1995)], et [2-O-(transféruloyl)-α-L-Araf (détermination de l'activité FA₂) isolés de pulpe de betterave [KROON et al., Carbohydrate Research, 300, 351-354, (1997)]. Le dosage est effectué à l'optimum des activités spécifiques des autres enzymes.

Le milieu réactionnel contient 100 µl de solution de substrat à 70 nmoles/L, 80 µl de tampon MOPS (3-N-morpholino-propane sulfonique) 0,1 mole/L, pH 6, et 20 µl de filtrat de culture. Le mélange est incubé 1 heure à 40°C. Aux temps : 0, 15, 30, 45, 60 minutes, 10 µl de milieu réactionnel sont prélevés et versés dans du tampon MOPS. Les densités optiques sont lues à 286 et 323 nm.

Le milieu réactionnel contient à la fois de l'acide férulique estérifié et de l'acide férulique libéré par l'enzyme. Leurs quantités respectives sont calculées à partir des densités optiques en utilisant les coefficients d'extinction molaires préalablement déterminés à pH 6 : ε₂₈₆ = 14176 L.mol⁻¹.cm⁻¹, ε₃₂₃ = 10350 L.mol⁻¹.cm⁻¹ pour l'acide férulique libre, et ε'₂₈₆ = 12465 L.mol⁻¹.cm⁻¹, ε'₃₂₃ = 19345 L.mol⁻¹.cm⁻¹ pour l'acide férulique estérifié.

Toutes les activités enzymatiques sont exprimées en nkat/mL, ce qui correspond à la quantité d'enzyme nécessaire à la libération d'une nmole de produit par seconde et par mL, dans les conditions de pH et de température définies ci-dessus.

### C) Résultats

La production d'enzymes dégradant les polysaccharides pariétaux a été suivie au cours de la culture d'*A. niger* sur pulpe de betterave et au cours de la culture témoin sur maltose. Les activités enzymatiques ont été mesurées comme décrit ci-dessus.

### 1) Enzymes de dépolymérisation et osidases

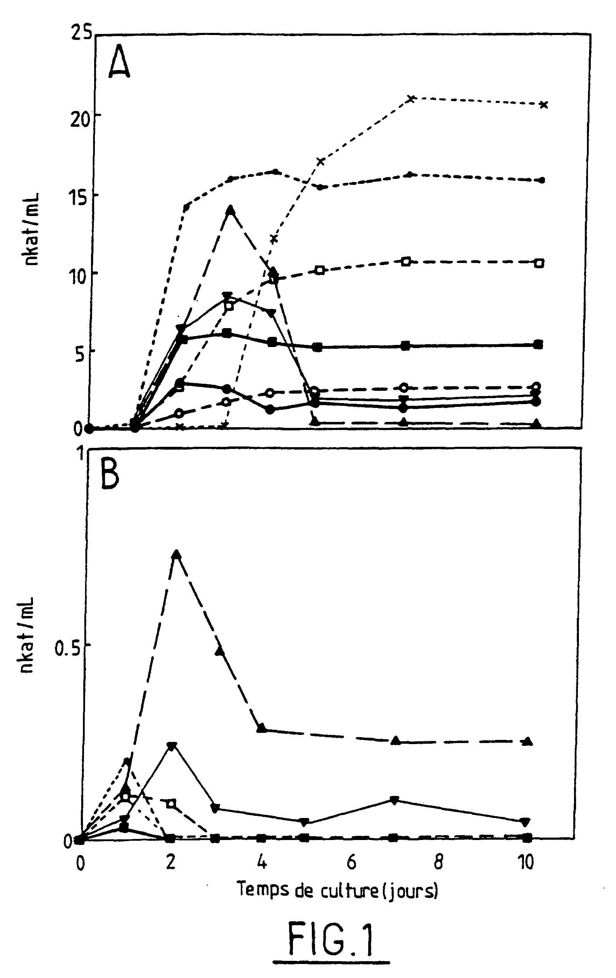

Les résultats obtenus pour les enzymes de dépolymérisation et les osidases sont illustrés par la figure 1, qui représente l'activité d'hydrolyse pour chaque substrat testé, en fonction du nombre de jours de culture.

### Légende de la Figure 1 :

- Figure 1A :: Induction sur pulpe de betterave ;
- Figure 1B :: Induction sur maltose ;
- ● :: Galactane ;
- ■ :: Arabinane ;
- ▲ :: Acide polygalacturonique ;
- ○ :: CarboxyMéthylCellulose ;
- □ :: Xylane ;
- ▼ :: Rhamnogalacturonane ;
- × :: pNPGalactoside ;
- • :: pNPArabinoside.

Lorsque le maltose seul est utilisé comme source carbonée dans les cultures d'*A. niger* I-1472, très peu d'activités enzymatiques actives sur les polysaccharides pariétaux sont libérées. Par contre, la présence de pulpe de betterave comme source carbonée inductrice conduit à une synthèse beaucoup plus importante d'enzymes dégradant les polysaccharides pariétaux. Un large spectre d'enzymes est présent dans le surnageant de culture. D'autre part, la synthèse de ces enzymes est rapide et l'on observe que les activités enzymatiques atteignent leur valeur maximum dès le 3ème jour de culture. Pour cette raison, les activités férulate estérases ont été recherchées dans les surnageants de culture à 3 jours.

### 2) Férulate estérases

Les activités férulate estérases dans les surnageants de culture sont indiquées dans le Tableau II suivant:

**Tableau II**

| Source carbonée inductrice | Activité (nkat/mL) | | |
|---|---|---|---|
| | FA | FAX | FA₂ |
| Témoin Maltose | 0 | 0 | 0 |
| Pulpe de betterave | 1,1 | 0,5 | 0 |

Une faible activité FA et FAX est donc induite lors de la culture d'*A. niger* I-1472 sur pulpe de betterave.

### EXEMPLE 2 : ACTIVITÉS ENZYMATIQDES D'A. niger I-1472 CULTIVÉ SUR HYDROLYSATS DE PULPE DE BETTERAVE

La pulpe de betterave subit deux hydrolyses successives par de l'acide trifluoroacétique pour en extraire les oligomères féruloylés, selon le protocole suivant [RALET et al. Carbohydrate Research, 263, 227-241 (1994)] :

800 g de pulpe de betterave sont mélangés à raison de 20 g/L à une solution d'acide trifluoracétique 50 mmoles/L. Le mélange est maintenu à 100°C pendant 1h30. La fraction soluble est recueillie, et précipitée par addition de 4 volumes d'éthanol. La fraction soluble dans l'éthanol est recueillie, et constitue l'hydrolysat 1.

Le précipité est traité par l'acide trifluoracétique (150 mmoles/L) pendant 6 heures à 100°C. La fraction soluble est recueillie, et précipitée par addition de 4 volumes d'éthanol. La fraction soluble dans l'éthanol est recueillie et constitue l'hydrolysat 2.

Les fractions notées : « hydrolysat 1 » et : « hydrolysat 2 » sont obtenues avec des rendements de 280 mg/g pour l'hydrolysat 1 et de 76 mg/g pour l'hydrolysat 2, et ont les compositions (poids sec) suivantes :

| Hydrolysat : | 1 | 2 |
|---|---|---|
| Rhamnose | 6 mg/g | 54 mg/g |
| Arabinose | 535 mg/g | 129 mg/g |
| Xylose | 2 mg/g | 2 mg/g |
| Galactose | 16 mg/g | 187 mg/g |
| Glucose | 85 mg/g | 42 mg/g |
| Acides uroniques | 17 mg/g | 188 mg/g |
| Acide férulique | 10 mg/g | 12 mg/g |
| Protéines | 12 mg/g | 8 mg/g |
| Cendres | 64 mg/g | 86 mg/g |

Les hydrolysats 1 ou 2 sont ensuite utilisés comme sources carbonées inductrices dans une culture *d'A. niger* I-1472, et les enzymes produites par le champignon sont mesurées selon les protocoles décrits à l'exemple 1.

### 1) Enzymes de dépolymérisation et osidases

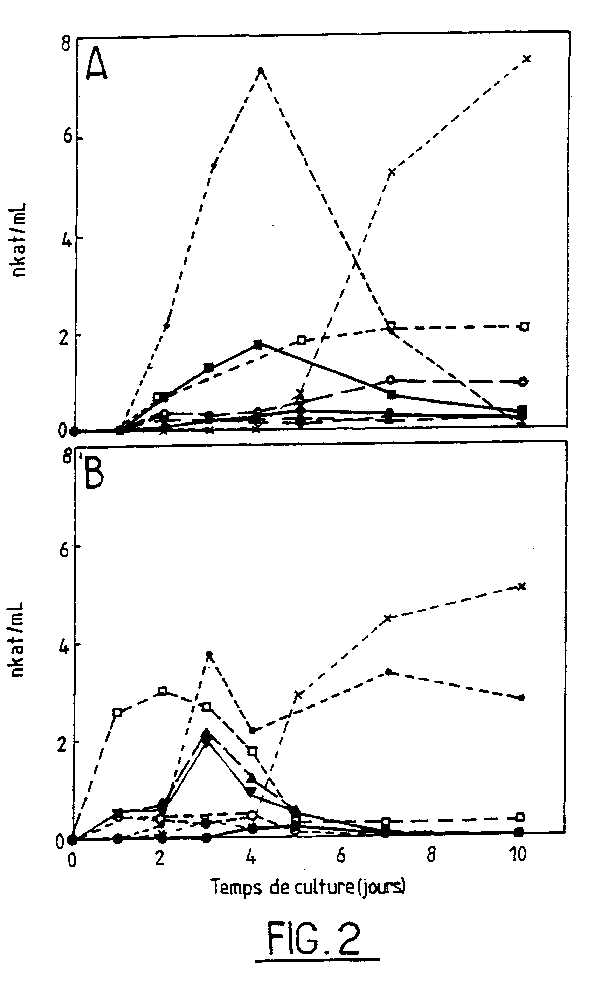

Les résultats obtenus pour les enzymes de dépolymérisation et les osidases sont illustrés par la figure 2 qui représente l'activité d'hydrolyse pour chaque substrat testé, en fonction du nombre de jours de culture.

### Légende de la Figure 2 :

- Figure 2A :: Induction sur hydrolysat 1;
- Figure 2B :: Induction sur hydrolysat 2 ;
- ● :: Galactane ;
- ■ :: Arabinane ;
- ▲ :: Acide polygalacturonique ;
- ○ :: CarboxyMéthylCellulose ;
- □ :: Xylane ;
- ▼ :: Rhamnogalacturonane ;
- × :: pNPGalactoside ;
- • :: pNPArabinoside.

Lorsque les hydrolysats 1 ou 2 de pulpe de betterave sont utilisés comme sources carbonées inductrices dans les cultures d'*A. niger* I-1472, la production d'enzymes dégradant les polysaccharides pariétaux est plus faible que dans les cultures sur pulpe de betterave. Les hydrolysats induisent donc la synthèse de quantités plus faibles d'enzymes. D'autre part, on observe que les activités enzymatiques atteignent leur valeur maximale après 4 jours de culture sur l'hydrolysat 1, et après 3 jours sur l'hydrolysat 2.

### 2) Férulate estérases

Les activités férulate estérases ont été mesurées dans les surnageants du 3ème jour de culture pour l'hydrolysat 2, et du 4ème jour de culture pour l'hydrolysat 1. Les résultats sont illustrés par le Tableau III ci-dessous.

**Tableau III**

| Source carbonée inductrice | Activité (nkat/mL) | | |
|---|---|---|---|
| | FA | FAX | FA₂ |
| Témoin Maltose | 0 | 0 | 0 |
| Hydrolysat 1 | 0,6 | 0 | 0,2 |
| Hydrolysat 2 | 0 | 0 | 0 |

De faibles activités FA et FA₂ sont induites lors de la culture d'*A. niger* I-1472 sur l'hydrolysat 1 de pulpe de betterave, riche en acide férulique estérifié à l'arabinose.

### EXEMPLE 3 : ACTIVITÉS ENZYMATIQUES PRODUITES PAR A. niger I-1472 CULTIVÉ SUR SON DE MAÏS

*A. niger* I-1472 est mis en culture en présence de son de maïs en tant que source carbonée inductrice, et les enzymes produites par le champignon sont mesurées, selon les protocoles décrits à l'exemple 1.

La composition du son de maïs (poids sec) est la suivante :

| | |
|---|---|
| Rhamnose | 0 mg/g |
| Arabinose | 154 mg/g |
| Xylose | 276 mg/g |
| Galactose | 51 mg/g |
| Glucose | 248 mg/g |
| Acides uroniques | 42 mg/g |
| Acide férulique | 31 mg/g |
| Protéines | 50 mg/g |

### 1) Enzymes de dépolymérisation et osidases

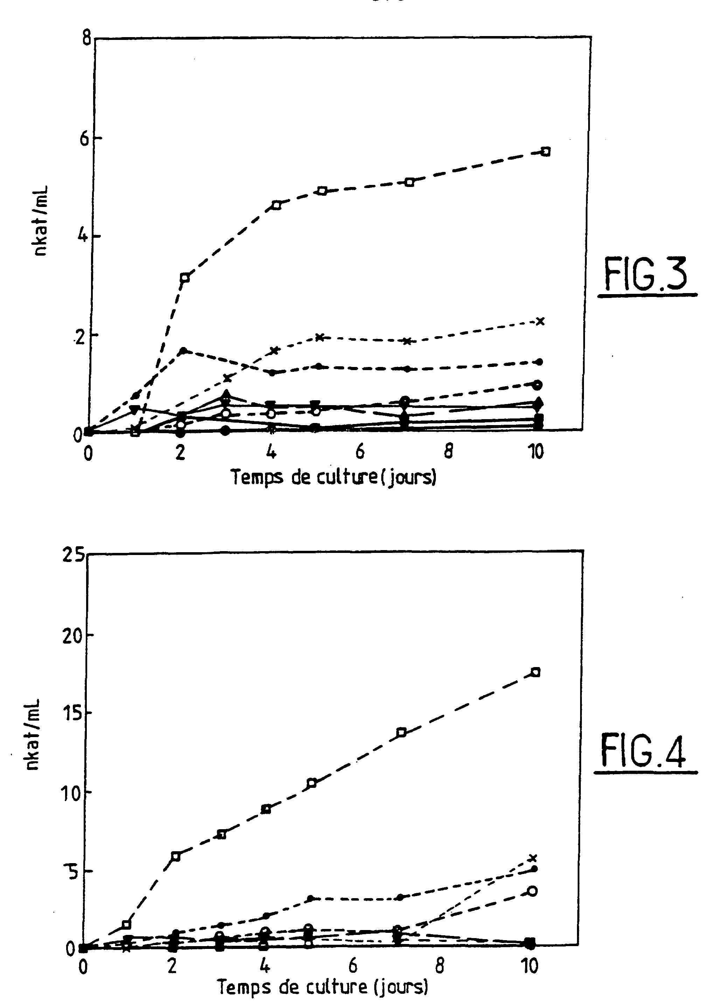

Les résultats obtenus pour les enzymes de dépolymérisation et les osidases sont illustrés par la figure 3, qui représente l'activité d'hydrolyse pour chaque substrat testé, en fonction du nombre de jours de culture.

### Légende de la Figure 3 :

- ● :: Galactane ;
- ■ :: Arabinane ;
- ▲ :: Acide polygalacturonique ;
- ○ :: CarboxyMéthylCellulose ;
- □ :: Xylane ;
- ▼ :: Rhamnogalacturonane ;
- × :: pNPGalactoside ;
- • :: pNPArabinoside.

Ces résultats montrent que dans le cas des enzymes de dépolymérisation et des osidases, les activités enzymatiques induites lors de la culture en présence de son de maïs sont plus faibles que celles induites en présence de pulpe de betterave.

### 2) Férulate estérases

Les activités férulate estérases mesurées dans le surnageant après 3 jours de culture sont respectivement de 4,6 nkat/mL pour FA et 4,9 nkat/mL pour FAX. On observe une nette induction des activités FA et FAX par le son de maïs.

### EXEMPLE 4 : ACTIVITÉS ENZYMATIQUES PRODUITES PAR A. NIGER I-1472 CULTIVÉ SUR L'AUTOCLAVAT ISSU DE SON DE MAÏS

Le son de maïs subit un traitement par autoclavage pour en extraire les oligomères féruloylés selon le protocole suivant :

Le son est mis en suspension dans l'eau (à raison de 100 g de son par litre), puis autoclavé à 160°C pendant 60 minutes. L'autoclavat est centrifugé pendant 10 minutes à 20 000 t/min, puis filtré sur verre fritté G3 (taille des pores 15-40 µm ; SCHOTT). Le surnageant filtré est lyophilisé. Le produit final, dénommé ci-après « autoclavat de son de maïs », est obtenu avec un rendement de 600 mg/g et a la composition (poids sec) suivante :

| | |
|---|---|
| Rhamnose | 0 mg/g |
| Arabinose | 208 mg/g |
| Xylose | 386 mg/g |
| Galactose | 73 mg/g |
| Glucose | 39 mg/g |
| Acides uroniques | 47 mg/g |
| Acide férulique | 34 mg/g |
| Protéines | 8 mg/g |
| Cendres | 8 mg/g |

L'autoclavat est ensuite utilisé comme source carbonée inductrice pour la culture d'*A. niger* I-1472 et les enzymes sont mesurées dans le surnageant de culture selon les protocoles décrits à l'exemple 1.

### 1) Enzymes de dépolymérisation et osidases

Les résultats obtenus pour les enzymes de dépolymérisation et les osidases sont illustrés par la figure 4 qui représente l'activité d'hydrolyse pour chaque substrat testé, en fonction du nombre de jours de culture.

### Légende de la Figure 4 :

- ● :: Galactane ;
- ■ :: Arabinane ;
- ▲ :: Acide polygalacturonique ;
- ○ :: CarboxyMéthylCellulose ;
- □ :: Xylane ;
- ▼ :: Rhamnogalacturonane ;
- × :: pNPGalactoside ;
- •· :: pNPArabinoside.

On constate en particulier que l'activité xylanase induite en présence d'hydrolysat de son de maïs est beaucoup plus élevée que celle induite lorsque le son de maïs natif est utilisé comme source carbonée inductrice.

### 2) Férulate estérases

Les activités férulate estérases mesurées dans le surnageant après 5 jours de culture, sont respectivement de 9,7 nkat/mL pour FA et de 10,6 nkat/mL pour FAX ; on observe donc une induction encore plus importante que celle observée dans le cas des cultures effectuées en présence de son de maïs.

### EXEMPLE 5 : LIBÉRATION D'ACIDE FÉRULIQUE PAR LES ENZYMES D'A. niger I-1472

Le surnageant de cultures d'*A. niger* I-1472 cultivées en présence de pulpe de betterave comme décrit à l'exemple 1 ci-dessus, et concentré 20 fois par lyophilisation, a été utilisé comme source d'enzymes pour libérer l'acide férulique présent soit dans la pulpe de betterave soit dans l'autoclavat de son de maïs.

Cette libération a été comparée avec la libération d'acide férulique par des enzymes commerciales : SP 584 (NOVO) dans le cas de la pulpe de betterave et NOVOZYM 342 (NOVO) dans le cas de l'autoclavat de son de maïs.

Le Tableau IV ci-dessous illustre la comparaison entre les activités enzymatiques présentes dans les 4 préparations d'enzymes utilisées :

**Tableau IV**

| Substrat | Activité spécifique (nkat/mg) | | | |
|---|---|---|---|---|
| | Surnageant d'*A. niger* 1-1472 sur pulpe de betterave | Surnageant d'*A*. *niger sur* autoclavat de son de maïs | SP 584 | NOVOZYM 342 |
| Arabinane | 120.6 | 1,9 | 291,1 | 7,2 |
| Xylane | 125,5 | 93,9 | 62.2 | 104,3 |
| Galactane | 32,9 | 1,8 | 943,9 | 2,8 |
| Rhamnogalacturonane | 53,4 | 4,9 | 256,7 | nd |
| CMC | 13,6 | 12,2 | 4,7 | 24,9 |
| Acide polygalacturonique | 86,0 | 1,5 | 2400,2 | 0,4 |
| pNP-Rha | 12,9 | nd | 0,2 | 0,0 |
| pNP-Gal | 19,8 | 3,8 | 84,8 | 0,0 |
| pNP-Ara | 266,6 | 27,5 | 619,5 | 0,3 |
| XFA | 9,2 | 85,9 | 0,1 | 0,8 |
| FA | 5,8 | 90,4 | 0,1 | 0,2 |
| FA2 | 2,4 | nd | 0,3 | nd |
| nd : non déterminé | | | | |

### A) Libération de l'acide férulique contenu dans la pulpe de betterave

La dégradation enzymatique de pulpe de betterave a été réalisée en présence de 10 mg de protéines (SP 584 ou enzymes d'*A. niger* I-1472) par g de pulpe sèche, soit 10 mg de protéines pour 8 mg d'acide férulique estérifié initialement présent dans la pulpe de betterave. Après 24 h d'hydrolyse, la quantité d'acide férulique libéré par SP 584 représente 50% de cette quantité initiale d'acide férulique, et la quantité d'acide férulique libéré par les enzymes d'*A. niger* I-1472 représente 40% de cette quantité initiale. Les enzymes sécrétées par *A. niger* I-1472 sont donc légèrement moins efficaces que SP 584 pour libérer l'acide férulique présent dans la pulpe de betterave.

### B) Libération de l'acide férulique contenu dans l'autoclavat de son de maïs

La dégradation enzymatique de l'autoclavat de son de maïs a été réalisée en présence de 10 mg de protéines (NOVOZYM 342 ou enzymes d'A. niger I-1472) par g d'autoclavat sec, soit 10 mg de protéines pour 34 mg d'acide férulique estérifié initialement présent dans l'autoclavat de son de maïs. Après 24 h d'hydrolyse, la quantité d'acide férulique libéré par NOVOZYM 342 représente 33% de cette quantité initiale d'acide férulique, et la quantité d'acide férulique libéré par les enzymes d'*A. niger* I-1472 représente 95% de cette quantité initiale. Les enzymes sécrétées par *A. niger* I-1472 sont donc beaucoup plus efficaces que NOVOZYM 342 pour libérer l'acide férulique contenu dans l'autoclavat de son de maïs.

### EXEMPLE 6 : BIOCONVERSION DIRECTE DE L'ACIDE FÉRULIQUE PRÉSENT DANS DES CO-PRODUITS AGRICOLES OU LEURS FRACTIONS, EN ACIDE VANILLIQUE PAR A. niger I-1472 CULTIVÉ EN PRÉSENCE DE SON DE MAÏS

*A. niger* I-1472 a été cultivé en présence de son de maïs comme source carbonée inductrice d'enzymes capables de dégrader les polysaccharides pariétaux. La production d'acide vanillique a été suivie en utilisant respectivement l'autoclavat de maïs, la pulpe de betterave ou ses hydrolysats comme substrat végétal source d'acide férulique.

### Conditions de bioconversion d'acide férulique en acide vanillique

La souche d'*A. niger* I-1472 a été cultivée dans les mêmes conditions de culture que celles décrites préalablement pour la production d'enzymes. Les cultures sont réalisées en bioréacteurs de laboratoire (2 litres de capacité) à agitation mécanique.

A l'optimum de production des enzymes, (en général au 3ème jour d'incubation de la culture), le substrat végétal source d'acide férulique estérifié est ajouté à la culture de *A. niger* I-1472 à raison d'une quantité correspondant à 0,3 à 1,5 g d'acide férulique par litre de culture et par jour.

La bioconversion de l'acide férulique libéré en acide vanillique est suivie par HPLC. L'analyse HPLC s'effectue sur des aliquots du milieu de culture prélevés à intervalles de temps réguliers et filtrés sur fibres de verre.

Les conditions d'analyse sont les suivantes : Colonne HPLC MERCK LICHROSPHER 100 RP18 (5 µm, 125 × 4 mm), maintenue à 30°C ; débit de 0,75 mL/minute ; détection UV à 280 nm.

Solvant d'élution : A : 0,01% d'acide acétique dans l'eau ; B : méthanol. Le profil d'élution est le suivant : 20% de solvant B pendant 4 minutes ; gradient linéaire de 20 à 40% de solvant B pendant 24 minutes ; 100% de solvant B pendant 2 minutes ; retour à 20% de solvant B et équilibrage de la colonne pendant 5 minutes.

Les résultats obtenus sont donnés dans le tableau V ci-dessous, à l'optimum de production d'acide vanillique à partir des différentes sources d'acide férulique utilisées. La quantité d'acide férulique consommé correspond à la différence entre la quantité totale d'acide férulique ajouté à la culture sous forme de substrat végétal, et la quantité totale d'acide férulique présent dans la culture au moment du dosage (il n'est pas possible de mesurer directement la quantité d'acide férulique libéré, du fait qu'en présence des cellules d'*A. niger*, celui-ci est immédiatement converti en acide vanillique au fur et à mesure de sa libération)

**Tableau V**

| Source d'acide férulique | Quantité totale d'acide férulique lié ajouté à la culture (mg/L) | Quantité d'acide férulique consommé (mg/L) | Acide vanillique produit (mg/L) |
|---|---|---|---|
| Son de maïs natif | 600 | 0 | 0 |
| Autoclavat de son de maïs | 4240 | 3460 | 1400 (après 7 jours de culture) |
| Pulpe de betterave | 600 | 0 | 0 |
| Hydrolysat 1 de pulpe de betterave | 1680 | 1160 | 350 (après 6 jours de culture) |
| Hydrolysat 2 de pulpe de betterave | 4240 | 3730 | 950 (après 7 jours de culture) |

On observe une production maximale d'acide vanillique lorsque les substrats végétaux riches en oligosaccharides féruloylés (hydrolysats de pulpe de betterave et autoclavat de son de maïs) servent de sources d'acide férulique. L'acide férulique est donc libéré par les enzymes induites de *A. niger* I-1472 et immédiatement biotransformé en acide vanillique par les enzymes intracellulaires, à raison de 1400 mg/L en 7 jours avec un rendement molaire de 46% par rapport à l'acide férulique consommé, et de 950 mg/L en 7 jours avec un rendement molaire de 29% par rapport à l'acide férulique consommé, lorsque l'autoclavat de son de maïs et l'hydrolysat 2 (riche en acide férulique estérifié au galactose) sont respectivement utilisés comme source d'acide férulique.

### EXEMPLE 7 : BIOCONVERSION DIRECTE DE L'ACIDE FÉRULIQUE PRÉSENT DANS DES CO-PRODUITS AGRICOLES OU LEURS FRACTIONS EN ACIDE VANILLIQUE PAR A. NIGER I-1472 CULTIVÉ EN PRÉSENCE DE PULPE DE BETTERAVE

Dans cet exemple, la source carbonée inductrice d'enzymes est la pulpe de betterave. Le son de maïs, l'autoclavat de maïs, la pulpe de betterave ou ses hydrolysats 1 ou 2 sont utilisés comme source d'acide férulique.

La production d'acide férulique et d'acide vanillique par *A. niger* I-1472 est suivie comme indiqué à l'exemple 6 ci-dessus.

Les résultats obtenus sont donnés dans le Tableau VI suivant, à l'optimum de production d'acide vanillique à partir des différentes sources d'acide férulique utilisées.

**Tableau VI**

| Source d'acide férulique | Quantité totale d'acide férulique lié ajoutée à la culture (mg/L) | Quantité totale d'acide férulique consommé (mg/L) | Acide vanillique produit (mg/L) |
|---|---|---|---|
| Son de maïs natif | 600 | 0 | 0 |
| Autoclavat de son de mais | 3750 | 3290 | 2200 (après 7 jours de culture) |
| Pulpe de betterave | 600 | 350 | 50 (après 6 jours de culture) |
| Hydrolysat 1 de pulpe de betterave | 900 | 650 | 150 (après 4 jours de culture) |
| Hydrolysat 2 de pulpe de betterave | 900 | 770 | 270 (après 4 jours de culture) |

Ces résultats montrent que l'utilisation de pulpe de betterave comme source carbonée inductrice conduit à des résultats de bioconversion directe meilleurs que ceux observés dans le cas de l'utilisation du son de mais comme source carbonée inductrice ; ceci est en accord avec les résultats sur les potentialités des enzymes de *A. niger* I-1472 induites dans ces conditions (exemples 1 et 2). En effet, dans le cas des cultures effectuées en présence de pulpe de betterave l'on observe pour les enzymes de dépolymérisation et les osidases, un spectre d'activités enzymatiques plus large et des niveaux d'activité plus élevés que dans le cas des cultures réalisées en présence de son de maïs ; ceci permet la libération d'une plus grande quantité et d'une plus grande variété d'oligosaccharides féruloylés utilisés comme substrat par les férulate estérases.

D'autre part, une production maximale d'acide vanillique est obtenue lorsque l'autoclavat de son de maïs est utilisé comme substrat végétal source d'acide férulique. Dans les cultures réalisées en présence de pulpes de betterave comme source carbonée inductrice des enzymes de dépolymérisation et des osidases, l'ajout d'autoclavat de maïs induit en outre les activités férulate estérases.

L'acide férulique libéré par les enzymes induites de *A. niger* I-1472 est ensuite très efficacement biotransformé en acide vanillique par les enzymes intracellulaires, à raison de 2200 mg/L en 7 jours avec un rendement molaire de 77% par rapport à l'acide férulique consommé.

Il est à noter que l'acide férulique lié présent dans les pulpes de betterave et leurs hydrolysats utilisés comme substrat végétal source d'acide férulique a également été bioconverti directement en acide vanillique, bien qu'à un niveau moindre.

### EXEMPLE 8 : BIOCONVERSION DIRECTE DE L'ACIDE FÉRULIQUE PRÉSENT DANS L'AUTOCLAVAT DE MAÏS EN ACIDE VANILLIQUE PAR A. NIGER I-1472 CULTIVÉ EN PRÉSENCE D'HYDROLYSATS DE PULPE DE BETTERAVE

Dans cet exemple, les sources carbonées inductrices d'enzymes sont les hydrolysats 1 et 2 de pulpe de betterave. L'autoclavat de maïs est utilisé comme substrat végétal source d'acide férulique

La production d'acide férulique et d'acide vanillique par *A. niger* I-1472 est suivie comme indiqué à l'exemple 6 ci-dessus.

Les résultats obtenus à l'optimum de production d'acide vanillique sont illustrés par le Tableau VII suivant :

**Tableau VII**

| Source carbonée inductrice | Quantité totale d'acide férulique lié ajoutée à la culture (mg/L) | Quantité totale d'acide férulique consommé (mg/L) | Acide vanillique produit (mg/L) |
|---|---|---|---|
| Hydrolysat 1 (riche en acide férulique estérifié à l'arabinose) | 3300 | 3150 | 1260 (après 7 jours de culture) |
| Hydrolysat 2 (riche en acide férulique estérifié au galactose) | 3300 | 3180 | 1550 (après 7 jours de culture) |

La production d'acide vanillique par A. niger I-1472 à partir de l'acide férulique contenu dans l'autoclavat de maïs est de 1260 mg/L après 7 jours (soit un rendement molaire de 46% par rapport à l'acide férulique consommé), lorsque l'hydrolysat 1 sert de source carbonée inductrice, et de 1550 mg/L après 7 jours (soit un rendement molaire de 56% par rapport à l'acide férulique consommé), lorsque l'hydrolysat 2 sert de source carbonée inductrice.

## Revendications

1. Procédé d'obtention de cultures d'*Aspergillus niger* à large spectre d'activité enzymatique comprenant des enzymes dégradant les polysaccharides pariétaux et des férulate estérases, lequel procédé est **caractérisé en ce qu'**il comprend la mise en culture d'au moins une souche d'*Aspergillus niger* en présence d'au moins une source carbonée inductrice choisie dans le groupe constitué par :
- la pulpe de betterave ou au moins une de ses fractions solubles riches en oligosaccharides féruloylés, susceptibles d'être obtenues par hydrolyse acide ;
- un son de céréale, notamment de maïs, ou un mélange de sons de différentes céréales, ou au moins une de ses fractions solubles riches en oligosaccharides féruloylés, susceptibles d'être obtenues par autoclavage dudit son ou dudit mélange.

2. Procédé selon la revendication 1, **caractérisé en ce que** la source carbonée inductrice est présente dans ledit milieu de culture à une concentration comprise entre 1 et 50 g/L et de préférence entre 2,5 et 30 g/L.

3. Procédé selon une quelconque des revendications 1 ou 2, **caractérisé en ce que** la culture d'*Aspergillus niger* comprend au moins la souche CNCM I-1472.

4. Procédé d'obtention d'une préparation enzymatique à large spectre d'activité comprenant des enzymes dégradant les polysaccharides pariétaux et des férulate estérases, **caractérisé en ce qu'**il comprend la mise en oeuvre du procédé selon une quelconque des revendications 1 à 3, et la récupération du surnageant de culture.

5. Préparation enzymatique, **caractérisée en ce qu'**elle est constituée par le surnageant d'une culture d'*Aspergillus niger* obtenue par le procédé selon une quelconque des revendications 1 à 3, ou par un concentré dudit surnageant.

6. Procédé de production d'acide férulique libre à partir d'un substrat féruloylé, lequel procédé est **caractérisé en ce qu'**il comprend la mise en contact dudit substrat avec au moins une culture d'*Aspergillus niger* obtenue par le procédé selon une quelconque des revendications 1 à 3, ou avec au moins une préparation enzymatique selon la revendication 5, dans des conditions permettant la libération de l'acide férulique par les enzymes présentes dans ladite culture ou ladite préparation enzymatique.

7. Procédé selon la revendication 6, **caractérisé en ce que** le substrat féruloylé est choisi parmi :
- la pulpe de betterave ou au moins une de ses fractions solubles riches en oligosaccharides féruloylés, susceptibles d'être obtenues par hydrolyse acide ;
- un son de céréale, notamment de maïs, ou un mélange de sons de différentes céréales, ou au moins une de ses fractions solubles riches en oligosaccharides féruloylés, susceptibles d'être obtenues par autoclavage dudit son ou dudit mélange.

8. Procédé selon une quelconque des revendications 6 ou 7, **caractérisé en ce que** l'on additionne au milieu de culture d'*Aspergillus niger* une quantité de substrat féruloylé correspondant à 0,1 à 50 g d'acide férulique par litre de milieu de culture.

9. Procédé selon une quelconque des revendications 6 ou 7, **caractérisé en ce que** l'on mélange la préparation enzymatique avec une quantité de substrat féruloylé correspondant à 0,1 à 40 g d'acide férulique pour 1 gramme de protéines totales de préparation enzymatique.

10. Procédé selon une quelconque des revendications 6 à 9, **caractérisé en ce que** la culture d'*Aspergillus niger* ou la préparation enzymatique sont produites en présence d'une source carbonée inductrice comprenant de la pulpe de betterave ou au moins une de ses fractions riches en oligosaccharides féruloylés susceptibles d'être obtenues par hydrolyse acide, et **en ce que** le substrat féruloylé comprend au moins un son de céréale ou au moins une de ses fractions riches en oligosaccharides féruloylés susceptibles d'être obtenues par autoclavage.

11. Procédé de production d'acide vanillique à partir d'un substrat féruloylé, **caractérisé en ce qu'**il comprend :
- la libération d'acide férulique à partir dudit substrat féruloylé, par une culture d'*Aspergillus niger* en mettant en oeuvre un procédé selon une quelconque des revendications 6 à 8, ou 10 ;
- la bioconversion en acide vanillique, par ladite culture d'*Aspergillus niger*, de l'acide férulique libéré du substrat féruloylé.

## Patentansprüche

1. Verfahren zum Erhalt von Kulturen von *Aspergillus niger* mit großem enzymatischen Aktivitätsspektrum, umfassend Enzyme, welche die parietalen Polysaccharide abbauen, und Ferulatesterasen, wobei das Verfahren **dadurch gekennzeichnet ist, dass** es Kultivieren mindestens eines *Aspergillus niger*-Stamms in Gegenwart mindestens einer induzierenden Kohlenstoffquelle, ausgewählt aus der Gruppe, bestehend aus:
- Rübenpulpe oder mindestens einer ihrer löslichen Fraktionen, die reich an feruloylierten Oligosacchariden sind, welche durch saure Hydrolyse erhalten werden können;
- einer Getreidekleie, insbesondere von Mais, oder einem Gemisch aus Kleien verschiedener Getreide oder mindestens einer ihrer löslichen Fraktionen, die reich an feruloylierten Oligosacchariden sind, die durch Behandlung der Kleie oder des Gemisches im Autoklaven erhalten werden können, umfasst.

2. Verfahren nach Anspruch 1, **dadurch gekennzeichnet, dass** die induzierende Kohlenstoffquelle im Kulturmedium mit einer Konzentration zwischen 1 und 50 g/l und vorzugsweise zwischen 2,5 und 30 g/l vorliegt.

3. Verfahren nach Anspruch 1 oder Anspruch 2, **dadurch gekennzeichnet, dass** die Kultur von *Aspergillus niger* mindestens den Stamm CNCM 1-1472 umfasst.

4. Verfahren zum Erhalt eines enzymatischen Präparats mit großem enzymatischen Aktivitätsspektrum, umfassend Enzyme, die die parietalen Polysaccharide abbauen, und Ferulatesterasen, **dadurch gekennzeichnet, dass** es die Durchführung des Verfahrens nach einem der Ansprüche 1 bis 3 und die Gewinnung des Kulturüberstandes umfasst.

5. Enzymatisches Präparat, **dadurch gekennzeichnet, dass** es aus dem Überstand einer Kultur von *Aspergillus niger,* der durch das Verfahren nach einem der Ansprüche 1 bis 3 erhalten wurde, oder aus einem Konzentrat des Überstands besteht.

6. Verfahren zur Herstellung von freier Ferulasäure, ausgehend von einem feruloylierten Substrat, wobei das Verfahren **dadurch gekennzeichnet ist, dass** es das Substrat mit mindestens einer Kultur von *Aspergillus niger,* erhalten durch das Verfahren nach einem der Ansprüche 1 bis 3, oder mit mindestens einem enzymatischen Präparat nach Anspruch 5 unter Bedingungen, welche die Freisetzung der Ferulasäure durch die Enzyme, die in der Kultur oder dem enzymatischen Präparat vorliegen, ermöglicht, umfasst.

7. Verfahren nach Anspruch 6, **dadurch gekennzeichnet, dass** das feruloylierte Substrat ausgewählt wird aus:
- Rübenpulpe oder mindestens einer ihrer löslichen Fraktionen, die reich an feruloylierten Oligosacchariden sind, welche durch saure Hydrolyse erhalten werden können;
- einer Getreidekleie, insbesondere von Mais, oder einem Gemisch aus Kleien verschiedener Getreide oder mindestens einer ihrer löslichen Fraktionen, die reich an feruloylierten Oligosacchariden sind, die durch Behandlung der Kleie oder des Gemisches im Autoklaven erhalten werden können.

8. Verfahren nach Anspruch 6 oder 7, **dadurch gekennzeichnet, dass** man dem Kulturmedium von *Aspergillus niger* eine Menge an feruloyliertem Substrat zusetzt, die 0,1 bis 50 g Ferulasäure pro Liter Kulturmedium entspricht.

9. Verfahren nach Anspruch 6 oder 7, **dadurch gekennzeichnet, dass** man das enzymatische Präparat mit einer Menge an feruloyliertem Substrat, die 0,1 bis 40 g Ferulasäure entspricht, pro 1 g Gesamtproteine des enzymatischen Präparats vermischt.

10. Verfahren nach einem der Ansprüche 6 bis 9, **dadurch gekennzeichnet, dass** die Kultur von *Aspergillus niger* oder das enzymatische Präparat in Gegenwart einer induzierenden Kohlenstoffquelle produziert wird, welche Rübenpulpe oder mindestens eine ihrer Fraktionen, die reich an feruloylierten Oligosacchariden sind, welche durch saure Hydrolyse erhalten werden können, umfasst, und dass das feruloylierte Substrat mindestens eine Getreidekleie oder mindestens eine ihrer Fraktionen, die reich an feruloylierten Oligosacchariden sind, welche durch Behandlung im Autoklaven erhalten werden können, umfasst.

11. Verfahren zur Herstellung von Vanillinsäure, ausgehend von einem feruloylierten Substrat, **dadurch gekennzeichnet, dass** es umfasst:
- die Freisetzung von Ferulasäure, ausgehend von dem feruloylierten Substrat, durch eine Kultur von *Aspergillus niger*, in dem ein Verfahren nach einem der Ansprüche 6 bis 8 oder 10 durchgeführt wird;
- die Biokonversion der aus dem feruloylierten Substrat freigesetzten Ferulasäure durch die Kultur von *Aspergillus niger* in Vanillinsäure.

## Claims

1. A process for producing *Aspergillus niger* cultures with a broad spectrum of enzymatic activity comprising enzymes which degrade the parietal polysaccharides and ferulate esterases, which process is **characterized in that** it comprises culturing at least one *Aspergillus niger* strain in the presence of at least one inducing carbon-containing source chosen from the group consisting of:
- beetroot pulp or at least a soluble fraction thereof rich in feruloylated oligosaccharides, which can be produced by acid hydrolysis;
- a cereal bran, in particular a maize bran, or a mixture of brans of various cereals, or at least a soluble fraction thereof rich in feruloylated oligosaccharides, which can be produced by autoclaving said bran or said mixture.

2. The process as claimed in claim 1, **characterized in that** the inducing carbon-containing source is present in said culture medium at a concentration of between 1 and 50 g/L, and preferably between 2.5 and 30 g/L.

3. The process as claimed in either of claims 1 or 2, **characterized in that** the *Aspergillus niger* culture comprises at least the CNCM 1-1472 strain.

4. A process for producing an enzymatic preparation with a broad spectrum of activity comprising enzymes which degrade the parietal polysaccharides and ferulate esterases, **characterized in that** it comprises carrying out the process as claimed in any one of claims 1 to 3, and recovering the culture supernatant.

5. An enzymatic preparation, **characterized in that** it consists of the supernatant of an *Aspergillus Niger* culture produced using the process as claimed in anyone of claims 1 to 3, or a concentrate of said supernatant.

6. A process for producing free ferulic acid from a feruloylated substrate, which process is **characterized in that** it comprises bringing said substrate into contact with at least one *Aspergillus niger* culture produced using the process as claimed in any one of claims 1 to 3, or with at least one enzymatic preparation as claimed in claim 5, under conditions which allow the release of the ferulic acid by the enzymes present in said culture or said enzymatic preparation.

7. The process as claimed in claim 6, **characterized in that** the feruloylated substrate is chosen from:
- beetroot pulp or at least a soluble fraction thereof rich in feruloylated oligosaccharides, which can be produced by acid hydrolysis;
- a cereal bran, in particular a maize bran, or a mixture of brans of various cereals, or at least a soluble fraction thereof rich in feruloylated oligosaccharides, which can be produced by autoclaving said bran or said mixture.

8. The process as claimed in either of claims 6 or 7, **characterized in that** an amount of feruloylated substrate corresponding to 0.1 to 50 g of ferulic acid per liter of culture medium is added to the *Aspergillus niger* culture medium.

9. The process as claimed in either of claims 6 or 7, **characterized in that** the enzymatic preparation is mixed with an amount of feruloylated substrate corresponding to 0.1 to 40 g of ferulic acid per gram of total proteins of the enzymatic preparation.

10. The process as claimed in any one of claims 6 to 9, **characterized in that** the *Aspergillus niger* culture or the enzymatic preparation is produced in the presence of an inducing carbon-containing source comprising beetroot pulp or at least a fraction thereof rich in feruloylated oligosaccharides, which can be produced by acid hydrolysis, and **in that** the feruloylated substrate comprises at least one cereal bran or at least a fraction thereof rich in feruloylated oligosaccharides, which can be produced by autoclaving.

11. A process for producing vanillic acid from a feruloulated substrate, **characterized in that** it comprises:
- the release of ferulic acid from said feruloylated substrate, by an *Aspergillus niger* culture by carrying out a process as claimed in anyone of claims 6 to 8, or 10;
- the bioconversion to vanillic acid, by said *Aspergillus niger*, of the ferulic acid released from the feruloylated substrate.
